# EUROPEAN PATENT SPECIFICATION

(11) **EP 1 100 788 B1**
(45) Date of publication and mention of the grant of the patent: **09.11.2005**
(21) Application number: 99939658.3
(22) Date of filing: 28.07.1999
(51) Int. Cl.: C07D 239/94, C07C 255/60, C07C 323/60

(54) **SUBSTITUTED QUINAZOLINE DERIVATIVES**
SUBSTITUIERTE CHINAZOLINE DERIVATE
DERIVES DE QUINAZOLINE SUBSTITUES

(30) Priority: 30.07.1998 US 126292
(43) Date of publication of application: 23.05.2001
(73) Proprietor: Wyeth, Madison, New Jersey 07940-0874 (US)
(72) Inventor: GLETSOS, Constantine, Pomona, NY 10970 (US)
(74) Representative: Mannion, Sally Kim
(86) International application number: PCT/US1999/017035
(87) International publication number: WO 2000/006555

(56) References cited:
- EP-A- 0 787 722

## Description

### BACKGROUND OF THE INVENTION

This invention relates to the preparation of certain quinazoline compounds as well as intermediates thereof. The quinazolines prepared by the process of the present invention inhibit the action of certain growth factor receptor protein tyrosine kinases (PTK) thereby inhibiting the abnormal growth of certain cell types. These quinazolines are anti-cancer agents and are useful for the treatment of cancer in mammals.

Protein tyrosine kinases are a class of enzymes that catalyze the transfer of a phosphate group from ATP to a tyrosine residue located on a protein substrate. Protein tyrosine kinases clearly play a role in normal cell growth. Many of the growth factor receptor proteins function as tyrosine kinases and it is by this process that they effect signaling. The interaction of growth factors with these receptors is a necessary event in normal regulation of cell growth. However, under certain conditions, as a result of either mutation or overexpression, these receptors can become deregulated; the result of which is uncontrolled cell proliferation which can lead to tumor growth and ultimately to the disease known as cancer [Wilks A.F., *Adv. Cancer Res., 60,* 43 (1993) and Parsons, J.T.; Parsons, S.J., *Important Advances in Oncology,* DeVita V.T. Ed., J.B. Lippincott Co., Phila., 3 (1993)]. Among the growth factor receptor kinases and their proto-oncogenes that have been identified and which are targets of the compounds of this invention are the epidermal growth factor receptor kinase (EGF-R kinase, the protein product of the erbB oncogene), and the product produced by the erbB-2 (also referred to as the neu or HER2) oncogene. Since the phosphorylation event is a necessary signal for cell division to occur and since overexpressed or mutated kinases have been associated with cancer, an inhibitor of this event, a protein tyrosine kinase inhibitor, will have therapeutic value for the treatment of cancer and other diseases characterized by uncontrolled or abnormal cell growth. For example, overexpression of the receptor kinase product of the erbB-2 oncogene has been associated with human breast and ovarian cancers [Slamon, D. J., et. al., *Science, 244,* 707 (1989) and *Science, 235 ,* 1146 (1987)]. Deregulation of EGF-R kinase has been associated with epidermoid tumors [Reiss, M., et. al., *Cancer Res., 51*, 6254 (1991)], breast tumors [Macias, A., et. al., *Anticancer Res., 7*, 459 (1987)], and tumors involving other major organs [Gullick, W.J., *Brit. Med. Bull*., *47,* 87 (1991)]. Because the importance of the role played by deregulated receptor kinases in the pathogenesis of cancer, many recent studies have dealt with the development of specific PTK inhibitors as potential anti-cancer therapeutic agents [some recent reviews: Burke. T.R., *Drugs Future*, *17,* 119 (1992) and Chang, C.J.; Geahlen, R.L., *J. Nat. Prod., 55,* 1529 (1992)].

### DESCRIPTION OF THE INVENTION

This invention provides a process for preparing a compound of formula 1: 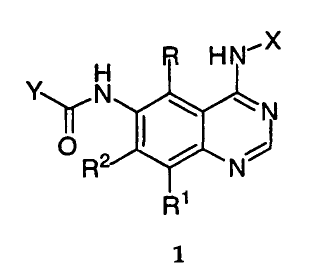 wherein:
- X: is phenyl optionally substituted with one or more substituents selected from the group consisting of halogen, alkyl of 1-6 carbon atoms, alkoxy of 1-6 carbon atoms, hydroxy, trifluoromethyl, cyano, nitro, carboxy, carboalkoxy of 2-7 carbon atoms, carboalkyl of 2-7 carbon atoms, amino, and alkanoylamino of 1-6 carbon atoms;
- R and R₁: are each, independently, hydrogen, halogen, alkyl of 1-6 carbon atoms, alkoxy of 1-6 carbon atoms, hydroxy, or trifluoromethyl;
- R₂: is hydrogen, alkyl of 1-6 carbon atoms, alkoxy of 1-6 carbon atoms, hydroxy, trifluoromethyl;
Y is 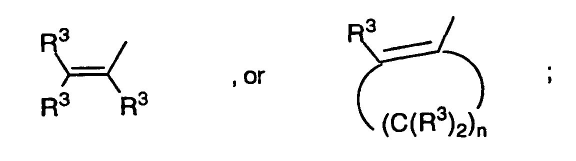
- R₃: is independently hydrogen, alkyl of 1-6 carbon atoms, carboxy, carboalkoxy of 1-6 carbon atoms, phenyl, or carboalkyl of 2-7 carbon atoms;
- n =: 2-4;
or a pharmaceutically acceptable salt thereof, which comprises:
a) acylating a compound of the formula: 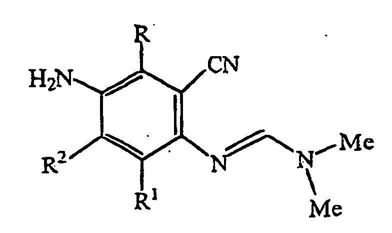 with an acid halide or mixed anhydride having the formula: 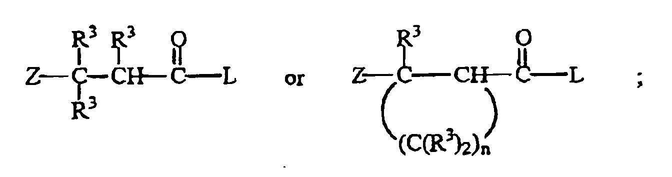 wherein
   Z is -OR⁴, -SR⁴, -SOR⁴, -SO₂R⁴, halogen, -NHR⁵, or -NR⁵R⁵;
   R⁴ is alkyl of 1-6 carbon atoms, cycloalkyl of 3-8 carbon atoms, or phenyl;
   R⁵ is alkyl of 1-6 carbon atoms or cycloalkyl of 3-8 carbon atoms;
   L is Cl, Br, or -OC(O)R⁶;
   R⁶ is alkyl of 1-6 carbon atoms, cycloalkyl of 3-8 carbon atoms, or phenyl;
   R³ and n are as defined above;
b) reacting the acylated product of step a) with H₂N-X,
   wherein
   X is as defined above; and
c) treating the compound of step b) under mild conditions using a base or Lewis acid to give the compound of Formula 1; and optionally
d) forming a pharmaceutically acceptable salt thereof.

The preparation of compounds of Formula 1 and use as antineoplastic agents have been disclosed in U.S. Patent 5,760,041. The processes described herein provide a new method of preparing these compounds which does not produce polymerization of the vinyl moiety (of the compounds Formula 1), which occurred using the procedures described in U.S. Patent 5,760,041.

The alkyl moieties described herein include both straight chain as well as branched carbon chains. Carboxy is defined as a -CO₂H radical. Carboalkoxy of 2-7 carbon atoms is defined as a -CO₂R" radical, where R" is an alkyl radical of 1-6 carbon atoms. Carboalkyl is defined as a -COR" radical, where R" is an alkyl radical of 1-6 carbon atoms. Halogen may be any suitable halogen, preferred halogens are fluorine, chlorine and bromine. When X is substituted, it is preferred that it is mono- , di- , or tri-substituted, with monosubstituted being most preferred. When X is substituted with halogen, it is preferably substituted with bromine. When a compound produced by the processes of this invention contains an assymetric center, this invention covers the individual R and S entantiomers as well as the racemate with respect to such compound. The pharmaceutically acceptable salts of compounds of Formula 1 are those derived from such organic and inorganic acids as: acetic, lactic, citric, tartaric, succinic, maleic, malonic, gluconic, hydrochloric, hydrobromic, phosphoric, nitric, sulfuric, methanesulfonic, and similarly known acceptable acids. The pharmaceutically acceptable salts can be prepared from the corresponding free base compounds using standard chemical methodology.

Of the compounds of this invention, preferred members include those in which R, R¹, and R² are hydrogen; and those in which R, R¹, and R² are hydrogen and X is either unsubstituted or monosubstituted with halogen, or alkyl of 1-6 carbon atoms. Preferred Z moieties include -OR⁴, -SR⁴, -SOR⁴, -SO₂R⁴, halogen, where R⁴ is alkyl of 1-6 carbon atoms. It is also preferred that the acylating agent has the formula:

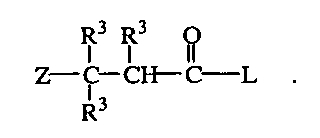

The following scheme illustrates the preparation of compounds of Formula 1; the preferred acylating agent is exemplified in this scheme. The starting materials used in this synthesis are either commercially available or can be prepared using standard chemical methodology. For example, the starting imino aniline can be prepared from the nitrophenyl imine disclosed in U.S. Patent 5,760,041.

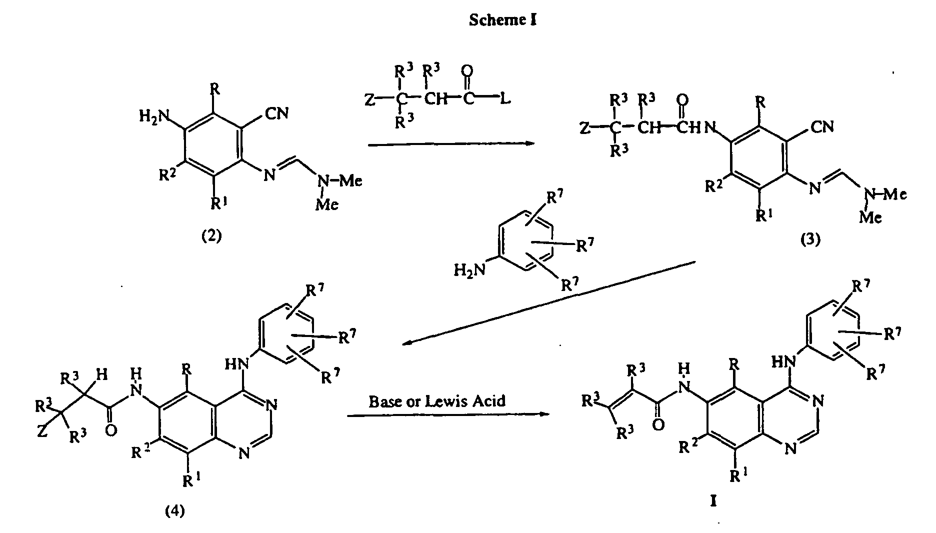

The acylating agent in the first step can either be an acid halide or can be a mixed anhydride. When the acylating agent is a mixed anhydride, the mixed anhydride can either be prepared as a separate step, or more preferably made in situ. The acylation reaction is typically carried out in the presence of a mild base, such as N-methyl morpholine, diisopropyl ethylamine, pyridine, and triethylamine.

In the second step, a Dimroth type rearrangement [Synthesis, 851(1988); Heterocyclic Chem., 16, 33(1974); Tetrahedron, 28, 535(1972); Z. Chem., 9, 241(1969)] can be carried out using a suitable solvent such as acetic acid, water, monohydric alcohols such as ethanol or isopropyl alcohol, or DMF, at temperatures ranging from ambient temperature to reflux. It is preferred that the reaction be carried out at temperatures above 78°C. Acetic acid at reflux were found to be the preferred conditions to effect this transformation.

Elimination of HZ to provide compounds of Formula I could be accomplished under mild conditions which prevented polymerization of the final product using bases such as potassium or sodium ethoxide, potassium or sodium t-butoxide, primary, secondary, or tertiary alkoxide bases, potassium or sodium carbonate in solvents such as ethanol, DMF, DMSO, THF, dioxane, methyl t-butyl ether, or diisopropyl ether. Acceptable reaction temperatures ranged from ambient to reflux. It is preferred that the elimination reaction be carried out using potassium t-butoxide in DMF at ambient temperature. It also preferred that at least three molar equivalents of base be used in this reaction. Alternatively, the elimination reaction could be accomplished using Lewis Acids such as ZnCl₂, nBu₄NF, CuSO₄, BF₃•Et₂O, or Yb(OTf)₃ in solvents such as nitrobenzene, nitromethane, carbon disulfide, or chlorinated hydrocarbons, such as dicloromethane or chloroform.

Scheme II shows the preparation of 4-(3-bromophenylamine)-6-(vinylamide)-quinazoline, a representative compound of Formula 1, using the methodology described above. The preparation and antineoplastic activity of 4-(3-bromophenylamine)-6-(vinylamide)quinazoline were disclosed in U.S. Patent 5,760,041.

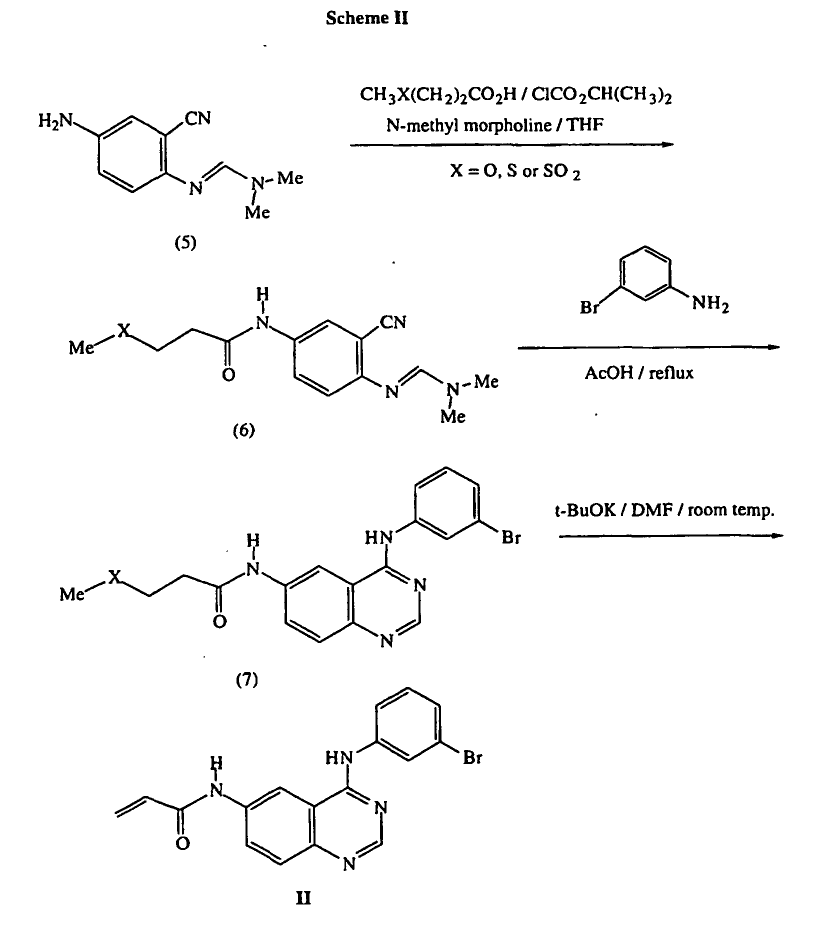

The first two steps of Scheme II can also be carried out as a one pot synthesis where X is O or S, thereby eliminating the need for isolation of compound (6).

Altematively, 4-(3-bromophenylamine)-6-(vinylamide)quinazoline was prepared by converting the methyl sulfide (7), the corresponding sulfoxide (12) or sulfone (13), followed by mild basic elimination of the better leaving group. Conversion to the sulfoxide was accomplished with one mole of m-chloroperbenzoic acid in THF/DMF at -50° C to ambient temperature. Other oxidants such as hydrogen peroxide and Caro's acid also provided satisfactory results. The corresponding sulfone was prepared from the methyl sulfide using OXONE (potassium peroxymonosulfate) in THF/methanol/water. The sulfone was also prepared using at least two molar equivalents of other oxidizing agents such as hydrogen peroxide, chlorine, ozone, or m-chloroperbenzoic acid. As 4-(3-bromophenylamine)-6-(vinylamide)quinazoline is useful as an antineoplastic agent, the compounds of Examples 1, 2, 4, 5, 7, 8, and 9 are useful as intermediates in its preparation.

The preparation of representative examples of the compounds covered by the process of this invention are described below.

### EXAMPLE 1

### 1-(3-methoxy propionyl)-3-cyano-4-(dimethyl formamidyl) aniline (6, X=O)

A solution of 3-methoxy propionic acid (1.30 g, 12 mmol) and isobutyl chloroformate (1.56 g, 12 mmol) in THF (20 mL) was stirred and cooled to 0 °C. To it were added dropwise over 30 min., a solution of N-methylmorpholine (1.21 g, 12 mmol) in THF (5 mL), while the temperature was maintained at 0-5 °C. The reaction mixture was stirred at 0-5 °C for another 15 min. and then to it was added over 30 min., a pre-made solution, prepared as follows: 3-cyano-4-(dimethylformamidyl) aniline (5) (2.26 g, 12 mmol) and N-methylmorpholine (1.21 g, 12 mmol) in THF (20 mL). This was a slurry and it had to be warmed to 35 °C to effect solubilization, before use. This solution maintained its clarity after cooling to 20-25 °C, before and during its addition. During the addition, the reaction temperature was maintained at 0-5 °C.

After the addition, the reaction mixture was stirred at 0-5 °C for 30 min.; the cooling was removed and the resulting slurry was stirred at 20-25 °C for another 2.5 hrs. An aqueous sodium chloride solution (20 mL, 20%) was added, the layers separated and the organic layer concentrated to provide an oil. Toluene (50 mL) was added to the oil and the mixture azeotroped in vacuo twice. Additional toluene (50 mL) was added and the mixture stirred, while cooling to 0-5 °C, the temperature was maintained at 0-5 °C until crystallization is established. The white solid was filtered and washed with toluene (3x5 mL) and hexanes (1x10 mL). The product was dried in a vacuum oven at 60 °C. A single crop of the title compound of 3.10 g (94.2%) was obtained; m.p. 143-145 °C..
TLC (dichloromethane-5% methanol) showed a single spot at R_{f} 0.36; ¹H NMR (300 MHz, DMSO-d₆) 2.52 (t, J=6Hz, 2H, CH₂-C=O), 2.97 (s, 3H, CH₃-N), 3.05 (s, 3H, CH₃-N), 3.24 (s, 3H, CH₃O-), 3.61 (t, J=6Hz, 2H, -CH₂-O), 7.90 (s, 1H, N=CH-N amidine), 7.1-8.0 (m, 3H, Ar-H), 10.05 (s, 1H, NH, amide).

### EXAMPLE 2

### 4-(3-bromo phenyl amine)-6-(3-methoxy propionyl amide)-quinazoline (7; X=O)

### {Direct method from (5), without isolation of (6; X=O) J

A solution of 3-methoxy propionic acid (1.30 g, 12 mmol) and isobutyl chloroformate (1.56 g, 12 mmol) in THF 20 mL) was stirred and cooled to 0 °C. To it was added, a solution of N-methylmorpholine (1.21 g, 12 mmol) in THF (5 mL), dropwise over 30 min. while maintaining 0-5 °C. The mixture was stirred at 0-5 °C for another 15 min. and then add to was added over 30 min., a pre-made solution prepared as follows: 3-cyano-4-(dimethylformamidyl) aniline (5) (2.26 g, 12 mmol) and N-methylmorpholine (1.21 g, 12 mmol) in THF (20 mL). This was a slurry and had to be warmed to 35 °C to effect solubilization, before use. This solution maintained its clarity after cooling to 20-25 °C, before and during its addition. During the addition, the reaction temperature was maintained at 0-5 °C.

After the addition, the mixture was stirred at 0-5 °C for 30 min., the cooling was removed, and the slurry was stirred at 20-25 °C for another 2.5 hrs. To it was added an aqueous sodium chloride solution (20%), the layers were separated and the organic layer concentrated in vacuo to an oil. Glacial acetic acid (15 mL) and 3-bromo aniline (2.29 g, 13.3 mmol) were added, and the mixture refluxed (114-116 °C) for 1.25 hrs.

The reaction mixture was concentrated in vacuo to a small volume (about 10 mL) and acetonitrile (30 mL) added. The mixture was heated to bring the oil into solution (about 60 °C) and then cooled slowly with slow stirring to induce crystallization (crystallization started at about 45 °C). The mixture was stirred at 20-25 °C for one hour, cooled to 0-5 °C and maintained for at least one additional hour. The solids were filtered and washed the with acetonitrile (4x 2 mL) and dried in a vacuum oven at 60 °C. to give 3.03 g (63.0%) of the title material as first crop; m.p. 216-218 °C; TLC and ¹H NMR shown below.

A second crop was obtained by basifying the filtrates with an aqueous sodium carbonate solution (20 mL, 20%), extracting with dichloromethane (2x20 mL), washing to neutrality with water (2x10 mL). The combined organic layers were concentrated under vacuum and azeotroped with toluene (2x20 mL) to a final volume (about 10 mL). Crystallization was initiated by stirring at 20-25 °C for at least one hour. The crystals were collected by filtration, washed with toluene (2x2 mL), hexanes (2x2 mL) and dried in a vacuum oven at 60 °C to give the title compound as a white solid (1.25 g; 26.1%), which was identical to the first crop; m.p. 216-218 °C.; TLC and ¹H NMR are shown below. Overall yield 4.28 g (89.1 %).
TLC (dichloromethane-5% methanol) showed a single spot at R_{f} 0.24; ¹H NMR (300 MHz, DMSO-d₆) 2.65 (t, J=6Hz, 2H, CH₂C=O), 3.28 (s, 3H, CH₃O-), 3.68 (t, J=6Hz, 2H, -CH₂O-), 7.20-8.80 (m, 8H, Ar-H), 9.95 (s, 1H, NH, amide), 10.35 (s, 1H, NH, amine).

### EXAMPLE 3

### 4-(3-bromo phenyl amine)-6-(vinyl amide) quinazoline (II) from (7, X=O)

To a solution of 4-(3-bromo phenyl amine)-6-(3-methoxy propionyl amide) quinazoline (7, X=O) (0.201 g, 0.5 mmol) in dimethyl formamide (2.5 mL), was added potassium *tert*-butoxide (0.178 g, 1.5 mmol). The mixture was stirred at ambient temperature for five hours, preferably overnight, until the reaction was completed (TLC/HPLC). A darkening and a small exotherm (34 °C) observed initially upon the base addition.

The mixture was partition between ethyl acetate (10 mL) and aqueous sodium chloride (10 mL, 20%). The layers were separated and the organic layer extracted to neutrality with aqueous sodium chloride (3x10 mL, 20%), dried over anhydrous magnesium sulfate (1.5 g) for at least one hour, and the slurry filtered over a plug of silica gel (3 g). The silica gel plug was washed with a solution of ethyl acetate-10% methanol (20 mL-2 mL), and the combined filtrates concentrated under vacuum to a yellow solid (0.200 g). Ethyl ether (5 mL) was added and the mixture stirred for one hour, filtered, and the solid washed with ethyl ether (2x0.5 mL) and dried under vacuum (60 °C) to provide the title compound as a light yellow solid, 0.154 g (83.4%); m.p. 288-290 °C(dec.).
TLC (dichloromethane-5% methanol) showed one single spot at R_{f} 0.40, identical to authentic;
¹H NMR (300 MHz, DMSO-d₆) 5.85 (d, J_{cis}=11Hz, 1H, C=CH geminal vinyl), 6.35 (d, Jₜᵣₐₙₛ=15Hz, 1H, C=CH geminal vinyl), 6.54 (q, Jₜᵣₐₙₛ=10Hz, J_{cis}=7Hz, 1H, C=CH-C=O- vicinal vinyl), 7.20-8.90 (m, 8H, Ar-H), 9.94 (s, 1H, NH amide), 10.55 (s, 1H, NH amine).

### EXAMPLE 4

### 1-(3-methylthio propionyl)-3-cyano-4-(dimethyl formamidyl) aniline (6; X=S)

A solution of 3-methylthio-propionic acid (2.00 g, 16.5 mmol) and isobutyl chloroformate (2.20 g, 16.5 mmol) in THF 25 mL) was stirred and cooled to 0 °C. To it was added dropwise over 30 min., a of solution of N-methylmorpholine (1.70 g, 16.5 mmol) in THF (5 mL), while maintaining a temperature of 0-5 °C. The mixture was stirred at 0-5 °C for another 15 min. and then over 30 min., was added a pre-made solution, prepared as follows: 3-cyano-4-(dimethylformamidyl) aniline(4) (3.10 g, 16.5 mmol) and N-methylmorpholine (1.70 g, 16.5 mmol) in THF (25 mL). This was a slurry and it had to be warmed to 35 °C to effect solubilization, before use. This solution maintained its clarity after cooling to 20-25 °C, before and during its addition. During the addition, the reaction mixture was maintained at 0-5 °C .

After the addition the mixture was stirred at 0-5 °C for 30 min.; the cooling was removed, and the slurry was stirred at 20-25 °C for another 2.5 hrs. An aqueous sodium chloride solution (25 mL, 20%) was added; the layers were separated and the organic layer concentrated in vacuo to an oil. Toluene (50 mL) was added to the oil and the mixture azeotroped in vacuo twice. Toluene (50 mL) was added to the glassy semi-solids; the mixture was heated to 60 °C and stirred for 15 min. The mixture was cooled to 20-25 °C and stirred for 0.5 hr., then cooled to 0-5 °C, and stirred for an additional 0.5 hr. The white solid was filtered and washed with toluene (3x10 mL) and hexanes (2x10 mL). The product was dried in a vacuum oven at 60 °C. A single crop of the title compound (3.80 g; 79.3%); m.p. 196-198 °C(dec.) was obtained.
TLC (dichloromethane-5% methanol) showed a single spot at R_{f} 0.48; ¹H NMR (300 MHz, DMSO-d₆) 2.09 (s, 3H, CH₃S-), 2.60 (t, J=7Hz, 2H, CH₂-C=O), 2.74 (t, J=7Hz, 2H, -CH₂-S), 2.98 (s, 3H, CH₃-N), 3.06 (s, 3H, CH₃-N), 7.93 (s, 1H, N=CH-N amidine), 7.1-8.0 (m, 3H, Ar-H), 10.15 (s, 1H, NH, amide).

### EXAMPLE 5

### 4-(3-bromo phenyl amine)-6-(3-methylthio propionyl amide) quinazoline (7; X=S)

### (Direct method from (5), without isolation of (6; X=S)}

A solution of 3-methylthio-propionic acid (2.00 g, 16.5 mmol) and isobutyl chloroformate (2.20 g, 16.5 mmol) in THF 25 mL) was stirred and cooled to 0 °C. To it was added a solution of N-methylmorpholine (1.70 g, 16.5 mmol) in THF (5 mL), dropwise over 30 min. while maintaining 0-5 °C. The mixture was stirred at 0-5 °C for another 15 min. and then the following solution was added to it over 30 min, while maintaining a reaction temperature of 0-5 °C: 3-cyano-4-(dimethylformamidyl) aniline (5) (3.10 g, 16.5 mmol) and N-methylmorpholine (1.70 g, 16.5 mmol) in THF (25 mL). This was a slurry and it had to be warmed to 35 °C to effect solubilization, before use. This solution maintained its clarity after cooling to 20-25 °C, before and during its addition.

After the addition the mixture was stirred at 0-5 °C for 30 min.; the cooling was removed and the slurry was stirred at 20-25 °C for another 2.5 hrs. An aqueous sodium chloride solution (25 mL, 20%) was added and the organic layer was separated and placed in a new flask. Glacial acetic acid (20 mL) was added to the organic layer, and the mixture concentrated under vacuum to a small volume (about 10 mL). To this syrupy solution was added glacial acetic acid (10 mL), 3-bromo aniline (3.10 g, 18.1 mmol), and the reaction mixture refluxed (114-116 °C) for 1.5 hr.

The clear solution was cooled to 20-25 °C and acetonitrile (40 mL) added. Crystallization started after cooling to 0-5 °C. The mixture was cooled for an additional hour at 0-5 °C. The white solid was filtered, washed with acetonitrile (3x5 mL) and dried under vacuum (60 °C.) to provide 4.38 g (63.6%) of the title compound, as a first crop; m.p. 236-239 °C; TLC and ¹H NMR are shown below.
A second crop was obtained by concentrating the filtrates to a small volume (about 10 mL), adding acetonitrile (40 mL), stirring at 20-25 °C for at least one hour and then at 0-5 °C for another hour. The resulting white solid was filtered, washed with acetonitrile (3x10 mL) and dried in the vacuum oven (60 °C) to provide 2.40 g (34.9%) as second crop, which was identical to the title compound; m.p. 236-239 °C.
Overall yield 6.78 g (98.5%).
TLC (dichloromethane-5% methanol) showed a single spot at R_{f} 0.20; ¹H NMR (300 MHz, DMSO-d₆) 2.12 (s, 3H, CH₃S-), 2.72 (t, J=6.5Hz, 2H, CH₂C=O), 2.81 (t, J=6.5Hz, 2H, -CH₂O-), 7.20-8.80 (m, 8H, Ar-H), 9.95 (s, 1H, NH, amide), 10.45 (s, 1H, NH, amine).

### EXAMPLE 6

### 4-(3-bromo phenyl amine)-6-(vinyl amide) quinazoline (II) from (7; X=S)

To a solution of 4-(3-bromo phenyl amine)-6-(3-methylthio propionyl amide) quinazoline (7, X=S) (0.208g, 0.5 mmol) in dimethyl formamide (2.5 mL), was added potassium *tert*-butoxide (0.178 g, 1.5 mmol) and the mixture was stirred at ambient temperature. A darkening and a small exotherm (34 °C) was observed initially upon the base addition. The reaction was progressing slowly at ambient temperature and it was not completed (TLC/HPLC) after two days.

The reaction mixture was partitioned between ethyl acetate (10 mL)/aqueous sodium chloride (10 mL, 20%). The layers were separated and the organic layer was extracted to neutrality with aqueous sodium chloride (3x10 mL, 20%). The organic layer was dried over anhydrous magnesium sulfate (1.5 g) for at least one hour and filtered over a plug of silica gel (3 g). The silica plug was washed with a solution of ethyl acetate-10% methanol (20 mL-2 mL). The filtrate was concentrated under vacuum to a yellow solid (0.184 g) and added to ethyl ether (5 mL). The mixture was stirred for one hour, filtered; the solid was washed with ethyl ether (2x0.5 mL) and dried under vacuum (60 °C). The light yellow solid, 0.150 g (81.2%) was a mixture of 50.5% the title compound (II) and 38.0% starting material (7, X=S) by HPLC. This was confirmed by TLC (dichloromethane-5% methanol) which showed two spots in a 50/40 ratio of (1)/(11) respectively. The ¹H NMR (300 MHz, DMSO-d₆) indicated the same ratio 5/4 of the characteristic frequencies of both compounds (II) and (7) respectively.

### EXAMPLE 7

### 1-(3-chloro propionyl)-3-cyano-4-(dimethyl formamidyl) aniline (Scheme I; 3, Z=Cl)

3-cyano-4-(dimethylformamidyl) aniline (5) (3.10 g, 16.5 mmol) and N-methylmorpholine (1.70 g, 16.5 mmol) was stirred in THF (25 mL). The mixture was a slurry and had to be warmed to 35 °C to effect solubilization. This solution lost its clarity after cooling and was maintained at 0-5 °C as a slurry.

To the above slurry was added very slowly, over 1.25 hr., a solution of 3-chloro-propionic acid (2.10 g, 16.5 mmol) in THF (10 mL). The reaction was maintained at 0-5 °C.; and stirred at that temperature for an additional half an hour. An aqueous sodium chloride solution (25 mL, 20%) was added, the layers separated and the organic layer concentrated in vacuo to a solid. Toluene (50 mL) was added to this solid and the mixture azeotroped in vacuo twice. Toluene (50 mL) was again added and the mixture heated to 60 °C, and stirred for 15 min. The mixture was cooled to 20-25 °C, stirred for 0.5 hr., then cooled to 0-5 °C, and stirred for an additional 0.5 hr. The resulting white solid was filtered and washed with toluene (2x25 mL) and ethyl ether (2x25 mL). The product was dried in a vacuum oven at 60 °C. A single crop of the title compound of 4.40 g (95.7%) was obtained; m.p. 174-175 °C. TLC (dichloromethane-5% methanol) showed a single spot at R_{f} 0.44; ¹H NMR (300 MHz, DMSO-d₆) 2.81 (t, J=6Hz, 2H, CH₂-C=O), 3.88 (t, J=6Hz, 2H, -CH₂-Cl), 2.98 (s, 3H, CH₃-N), 3.06 (s, 3H, CH₃-N), 7.92 (s, 1H, N=CH-N amidine), 7.1-8.0 (m, 3H, Ar-H), 10.20 (s, 1H, NH, amide).

### EXAMPLE 8

### 4-(3-bromo phenyl amine)-6-(3-methylsulfoxido propionyl amide)-quinazoline (12), from (7; X=S)

A solution of 4-(3-bromo phenyl amine)-6-(3-methylthio propionyl amine)-quinazoline (7; X=S, 0.208 g, 0.5 mmol), THF (2 mL) and DMSO (0.25 mL) was cooled to -50 °C. A solution of m-chloroperbenzoic acid (0.110 g, 0.5 mmol) in THF (1 mL) was added over one minute, keeping -40 to -50 °C. The reaction mixture was stirred at -50 °C for 30 minutes; the cooling removed and the mixture was allowed to warm to 20-25 °C.

The reaction mixture was partitioned between ethyl acetate (10 mL) and aqueous NaCl (10 mL, 20%). The organic layer was separated and extracted to neutrality with aqueous NaCl (3x10 mL) and water (1x10 mL).The organic layer was dried with MgSO₄ (1 g), filter, washed with ethyl acetate (3x2 mL) and concentrated under vacuum to a gray solid, which was triturated with acetonitrile (3 mL) by heating to 70 °C and then cooling to 20-25 °C. The solid was filtered, washed with acetonitrile (3x0.5 mL) and dried in a vacuum oven (60 °C). White solid identical to title compound (12) 0.203 g (93.8%); m.p. 286-288 °C(dec.).
TLC (dichloromethane-5% methanol) showed a single spot at R_{f} 0.10; ¹H NMR (300 MHz, DMSO-d₆) 2.61 (s, 3H, CH₃SO-), 2.88 (m, 2H, CH₂C=O), 3.16 (m, 2H, - CH₂S=O), 7.20-8.80 (m, 8H, Ar-H), 9.94 (s, 1H, NH, amide), 10.49 (s, 1H, NH, amine).

### EXAMPLE 9

### 4-(3-bromo phenyl amine)-6-(3-methylsulfono propionyl amide)-quinazoline (13), from (7; X=S)

To a solution of 4-(3-bromo phenyl amine)-6-(3-methylthio propionyl amine)-quinazoline (11; X=S, 0.208 g, 0.5 mmol), THF (2 mL) and MeOH (1 mL) cooled to 0 °C, was added add a solution of OXONE (0.616 g, 1 mmol) in water (2 mL) over one minute, keeping the reaction temperature at 0-5 °C. The mixture was stirred at 0-5 °C for 4.5 hours; the cooling was removed and the reaction allowed to warm to 20-25 °C.

The reaction mixture was partitioned between ethyl acetate (10 mL) and aqueous Na₂CO₃ (10 mL, 20%) resulting in an emulsion. The emulsion was diluted with aqueous NaCl (20 mL, 20%) and filtered. The solids were slurried and washed on a filter funnel with water (3x10 mL) and ethyl acetate (2x2 mL). The product was dried in a vacuum oven (60 °C). White solid consistent to title compound(13) 0.148g (65.9%); m.p. 248-250 °C(dec.).
TLC (dichloromethane-5% methanol) showed a single spot at R_{f} 0.36; ¹H NMR (300 MHz, DMSO-d₆) 2.92 (t, J=7.5Hz, 2H, CH₂C=O), 3.07 (s, 3H, CH₃SO₂-), 3.45 (t, J=7.5Hz, 2H, -CH₂SO₂), 7.20-8.80 (m, 8H, Ar-H), 9.95 (s, 1H, NH, amide), 10.49 (s, 1H, NH, amine).

### EXAMPLE 10

### 4-(3-bromo phenyl amine)-6-(3-methylsulfono propionyl amide) quinazoline (13), from (12)

To a solution of 4-(3-bromo phenyl amine)-6-(3-methysulfoxido propionyl amine) quinazoline (12, 0.216 g, 0.5 mmol), THF (3 mL), MeOH (2 mL) and DMF (2 mL) at 0 °C, was added add a solution of OXONE (0.616 g, 1 mmol) in water (2 mL) over one minute, while maintaining the temperature at 0-5 °C. The mixture was stirred at 0-5 °C for 0.5 hr. and at 20-25 °C for 2.5 hrs.

The reaction mixture was partitioned between ethyl acetate (10 mL) and aqueous Na₂CO₃ (10 mL, 20%). An emulsion resulted. The emulsion was diluted with aqueous NaCl (20 mL, 20%) and filtered. The solids were slurried and washed on a filter with water (2x2.5 mL) and ethyl acetate (2x2.5 mL). The product was dried in a vacuum oven (60 °C). The resulting white solid was consistent to title compound (13) 0.50g (67.0%); m.p. 248-250 °C(dec.).
TLC (dichloromethane-5% methanol) showed a single spot at R_{f} 0.36; ¹H NMR (300 MHz, DMSO-d₆) 2.92 (t, J=7.5Hz, 2H, CH₂C=O), 3.07 (s, 3H, CH₃SO₂-), 3.45 (t, J=7.5Hz, 2H, -CH₂SO₂), 7.20-8.80 (m, 8H, Ar-H), 9.95 (s, 1H, NH, amide), 10.49 (s, 1H, NH, amine).

### EXAMPLE 11

### 4-(3-bromo phenyl amine)-6-(vinyl amide) quinazoline (II) from (12)

To a solution of 4-(3-bromo phenyl amine)-6-(3-methylsulfoxido propionyl amide) quinazoline (12) (0.043g, 0.1 mmol) in DMF (2 mL), was added potassium *tert*-butoxide (0.035 g, 0.3 mmol). The reaction mixture was stirred at ambient temperature for one hour, whereupon the reaction was completed (TLC/HPLC). A darkening, but no exotherm was observed initially upon the base addition.

The reaction mixture was partitioned between ethyl acetate (10 mL) and aqueous sodium chloride (10 mL, 20%). The layers were separated, the organic layer extracted to neutrality with aqueous sodium chloride (3x10 mL, 20%), dried over anhydrous magnesium sulfate (1.5 g) for at least one hour, and filtered over a plug of silica gel (3 g). The plug was washed with a solution of ethyl acetate-10% methanol (20 mL-2 mL). The filtrates were concentrated under vacuum to a yellow solid (0.025 g). Ethyl ether (3 mL) was added, the mixture stirred for one hour, filtered, washed with ethyl ether (3x0.5 mL) and dried it under vacuum (60 °C). The resulting light yellow solid, 0.020 g (55.0%) was identical to the title compound (II); m.p. 288-290 °C(dec.).
TLC (dichloromethane-5% methanol) showed one single spot at R_{f} 0.40, identical to authentic;
¹H NMR (300 MHz, DMSO-d₆) 5.85 (d, J_{cis}=11Hz, 1H, C=CH geminal vinyl), 6.35 (d, Jₜᵣₐₙₛ=15Hz, 1H, C=CH geminal vinyl), 6.54 (q, Jₜᵣₐₙₛ=10Hz, J_{cis}=7Hz, 1H, C=CH-C=O- vicinal vinyl), 7.20-8.90 (m, 8H, Ar-H), 9.94 (s, 1H, NH amide), 10.55 (s, 1H, NH amine).

### EXAMPLE 12

### 4-(3-bromo phenyl amine)-6-(vinyl amide) quinazoline (II), from (13)

To a solution of 4-(3-bromo phenyl amine)-6-(3-methylsulfono propionyl amide) quinazoline (13) (0.090g, 0.1 mmol) in DMF (1.5 mL), was added potassium *tert*-butoxide (0.035 g, 0.3 mmol). The reaction mixture was stirred at ambient temperature for 2 hours, whereupon the reaction was completed (TLC/HPLC). A darkening and a slight exotherm (22 to 24 °C) was observed initially upon the base addition.

The mixture was partitioned between ethyl acetate (10 mL) and aqueous sodium chloride (10 mL, 20%). The layers were separated; the organic layer extracted to neutrality with aqueous sodium chloride (3x10 mL, 20%), dried over anhydrous magnesium sulfate (1.5 g) for at least one hour and filtered over a plug of silica gel (3 g). The plug was washed with a solution of ethyl acetate-10% methanol (20 mL-2 mL). The combined filtrates were concentrated under vacuum to a yellow solid (0.075 g) which was added to it ethyl ether (3 mL), stirred for one hour, filtered, washed with ethyl ether (3x0.5 mL) and dried under vacuum (60 °C). The light yellow solid, 0.055 g (74.3%) was identical to the title compound (1); m.p. 288-290 °C(dec.).
TLC (dichloromethane-5% methanol) showed one single spot at R_{f} 0.40, identical to authentic;
¹H NMR (300 MHz, DMSO-d₆) 5.85 (d, J_{cis}=11Hz, 1H, C=CH geminal vinyl), 6.35 (d, Jₜᵣₐₙₛ=15Hz, 1H, C=CH geminal vinyl), 6.54 (q, Jₜᵣₐₙₛ=10Hz, J_{cis}=7Hz, 1H, C=CH-C=O- vicinal vinyl), 7.20-8.90 (m, 8H, Ar-H), 9.94 (s, 1H, NH amide), 10.55 (s, 1H, NH amine).

## Claims

1. A process for preparing a compound of formula 1: 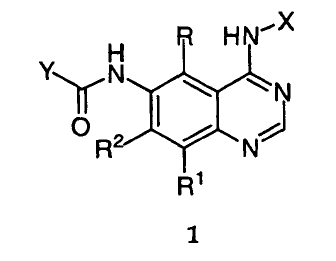 wherein:
X is phenyl optionally substituted with one or more substituents selected from the group consisting of halogen, alkyl of 1-6 carbon atoms, alkoxy of 1-6 carbon atoms, hydroxy, trifluoromethyl, cyano, nitro, carboxy, carboalkoxy of 2-7 carbon atoms, carboalkyl of 2-7 carbon atoms, amino, and alkanoylamino of 1-6 carbon atoms;
R and R₁ are each, independently, hydrogen, halogen, alkyl of 1-6 carbon atoms, alkoxy of 1-6 carbon atoms, hydroxy, or trifluoromethyl;
R₂ is hydrogen, alkyl of 1-6 carbon atoms, alkoxy of 1-6 carbon atoms, hydroxy, trifluoromethyl;
Y is 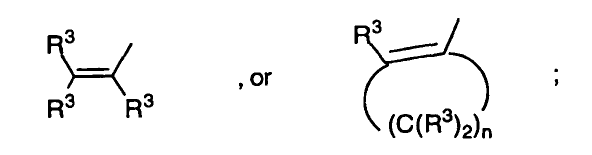
R₃ is independently hydrogen, alkyl of 1-6 carbon atoms, carboxy, carboalkoxy of 1-6 carbon atoms, phenyl, or carboalkyl of 2-7 carbon atoms;
n = 2-4;
or a pharmaceutically acceptable salt thereof, which comprises:
a) acylating a compound of the fonnula: 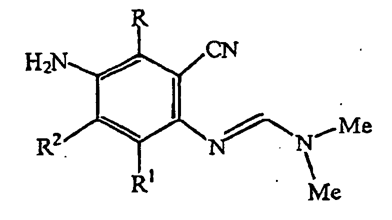 with an acid halide or mixed anhydride having the formula: 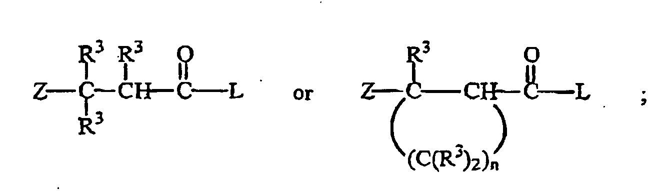 wherein
Z is -OR⁴, -SR⁴, -SOR⁴, -SO₂R⁴, halogen, -NHR⁵, or -NR⁵R⁵;
R⁴ is alkyl of 1-6 carbon atoms, cycloalkyl of 3-8 carbon atoms, or phenyl;
R⁵ is alkyl of 1-6 carbon atoms or cycloalkyl of 3-8 carbon atoms;
L is Cl, Br, or -OC(O)R⁶;
R⁶ is alkyl of 1-6 carbon atoms, cycloalkyl of 3-8 carbon atoms, or phenyl;
R³ and n are as defined above;
b) reacting the acylated product of step a) with H₂N-X,
wherein
X is as defined above; and
c) treating the compound of step b) under mild conditions using a base or Lewis acid to give the compound of Formula 1; and optionally
d) forming a phanmaceutically acceptable salt thereof.

2. The process according to claim 1 wherein the acylating agent of step a) is

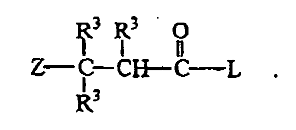

3. The process according to claim 2, wherein R, R¹, and R² are hydrogen.

4. The process according to claim 3, wherein X is either unsubstituted or is monosubstituted with halogen or alkyl of 1-6 carbon atoms.

5. The process according to claim 4, wherein Z is -OR⁴, -SR⁴, -SOR⁴, -SO₂R⁴, halogen and R⁴ is alkyl of 1-6 carbon atoms.

6. The process according to any of the preceding claims wherein steps a) and b) are carried out in a one pot synthesis.

7. A compound which is selected from the group consisting of 1-(3-methoxy propionyl)-3-cyano-4-(dimethyl formamidyl) aniline; 1-(3-methylthio propionyl)-3-cyano-4-(dimethyl formamidyl) aniline; and 1-(3-chloro propionyl)-3-cyano-4-(dimethyl formamidyl) aniline.

8. A compound which is selected from the group consisting of 4-(3-bromo phenyl amine)-6-(3-methoxy propionyl amide)-quinazoline; 4-(3-bromo phenyl amine)-6-(3-methylthio propionyl amide) quinazoline; 4-(3-bromo phenyl amine)-6-(3-methylsulfoxido propionyl amide)-quinazoline; and 4-(3-bromo phenyl amine)-6-(3-methylsulfono propionyl amide)-quinazoline.

## Patentansprüche

1. Verfahren zur Herstellung einer Verbindung der Formel 1: 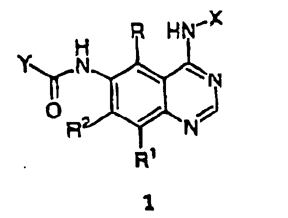 worin:
X für Phenyl steht, das gegebenenfalls mit einem oder mehreren Substituenten substituiert ist, die ausgewählt sind unter Halogen, Alkyl mit 1-6 Kohlenstoffatomen, Alkoxy mit 1-6 Kohlenstoffatomen, Hydroxy, Trifluormethyl, Cyano, Nitro, Carboxy, Carboalkoxy mit 2-7 Kohlenstoffatomen, Carboalkyl mit 2-7 Kohlenstoffatomen, Amino und Alkanoylamino mit 1-6 Kohlenstoffatomen;
R und R₁ jeweils unabhängig voneinander für Wasserstoff, Halogen, Alkyl mit 1-6 Kohlenstoffatomen, Alkoxy mit 1-6 Kohlenstoffatomen. Nydroxy oder Trifluormethyl stehen;
R₂ für Wasserstoff, Alkyl mit 1-6 Kohlenstoffatomen, Alkoxy mit 1-6 Kohlenstoffatomen, Hydroxy, Trifluormethyl steht;
Y für 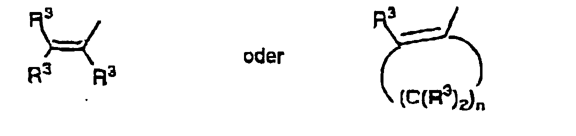 steht;
R₃ unabhängig für Wasserstoff, Alkyl mit 1-6 Kohlenstoffatomen. Carboxy, Carboalkoxy mit 1-6 Kohlenstoffatomen, Phenyl oder Carboalkyl mit 2-7 Kohlenstoffatomen steht;
n = 2-4;
oder eines pharmazeutisch akzeptablen Salzes davon, wobei man:
a) eine Verbindung der Formel 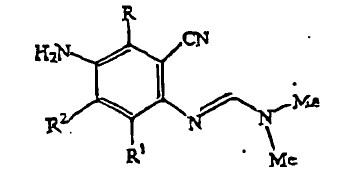 mit einem Säurehalogenid oder einem gemischten Anhydrid der Formel: 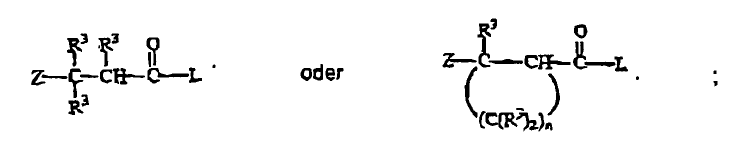 worin
Z für -OR⁴, -SR⁴, -SOR⁴, -SO₂R⁴. Halogen, -NHR⁵ oder -NR⁵R⁵ steht;
R⁴ für Alkyl mit 1-6 Kohlenstoffatomen, Cycloalkyl mit 3-8 Kohlenstoffatomen oder Phenyl steht;
R⁵ für Alkyl mit 1-6 Kohlenstoffatomen oder Cycloalkyl mit 3-8 Kohlenstoffatomen steht;
L für Cl, Br oder -OC(O)R⁵ steht;
R⁶ für Alkyl mit 1-6 Kohlenstoffatomen, Cycloalkyl mit 3-8 Kohlenstoffatomen oder Phenyl steht;
R³ und n wie oben definiert sind;
acyliert;
b) das acylierte Produkt aus Stufe a) mit H₂N-X umsetzt, wobei X wie oben definiert ist: und
c) die Verbindung aus Stufe b) unter milden Bedingungen unter Verwendung einer Base oder Lewis-Säure behandelt, wobei man die Verbindung der Formel 1 erhält; und gegebenenfalls
d) ein pharmazeutisch akzeptables Salz davon bildet

2. Verfahren nach Anspruch 1. wobei es sich bei dem Acylierungsmittel der Stufe a) um 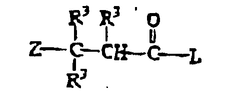 handelt.

3. Verfahren nach Anspruch 2, wobei R, R¹ und R² für Wasserstoff stehen.

4. Verfahren nach Anspruch 3, wobei X entweder unsubstituiert oder mit Halogen oder Alkyl mit 1-6 Kohlenstoffatomen monosubstituiert ist.

5. Verfahren nach Anspruch 4, wobei Z für -OR⁴, -SR⁴, -SOR⁴, -SO₂R⁴ steht, Halogen und R⁴ für Alkyl mit 1-6 Kohlenstoffatomen steht.

6. Verfahren nach einem der vorhergehenden Ansprüche, wobei die Stufen a) und b) in einer Ein-Topf-Synthese durchgeführt werden.

7. Verbindung, ausgewählt unter 1-(3-Methoxypropionyl)-3-cyano-4-(dimethylformmidyl)anilin; 1-(3-Methylthiopropionyl)-3-cyano-4-(dimethylformamidyl)anilin; und 1-(3-Chlorpropionyl)-3-cyano-4-(dimethylformamidyl)anilin.

8. Verbindung, ausgewählt unter 4-(3-Bromphenylamin)-6-(3-methoxypropionylamid)-chinazolin; 4-(3-Bromphenylamin)-6-(3-methylthiopropionylamid)chinazolin; 4-(3-Bromphenylamin)-6-(3-methylsulfoxidopropionylamid)chinazolin; und 4-(3-Bromphenylamin)-6-(3-methylsulfonopropionylamid)chinazolin.

## Revendications

1. Procédé pour la préparation d'un composé de formule 1 : 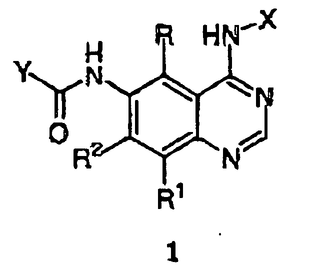 dans laquelle :
X représente un groupe phényle facultativement substitué avec un ou plusieurs substituants choisis dans le groupe consistant en des substituants halogéno, alkyle ayant 1 à 6 atomes de carbone, alkoxy ayant 1 à 6 atomes de carbone, hydroxy, trifluorométhyle, cyano, nitro, carboxy, carboalkoxy ayant 2 à 7 atomes de carbone, carboalkyle ayant 2 à 7 atomes de carbone, amino et alcanoylamino ayant 1 à 6 atomes de carbone ;
R et R₁ représentent chacun, indépendamment, un atome d'hydrogène, un atome d'halogène, un groupe alkyle ayant 1 à 6 atomes de carbone, alkoxy ayant 1 à 6 atomes de carbone, hydroxy ou trifluorométhyle ;
R₂ représente un atome d'hydrogène, un groupe alkyle ayant 1 à 6 atomes de carbone, alkoxy ayant 1 à 6 atomes de carbone, hydroxy ou trifluorométhyle ;
Y représente 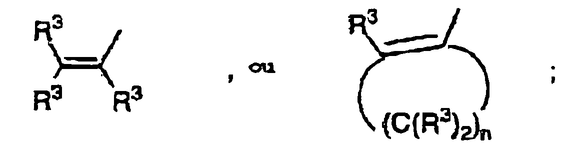
R₃ représente indépendamment un atome d'hydrogène, un groupe alkyle ayant 1 à 6 atomes de carbone, carboxy, carboalkoxy ayant 1 à 6 atomes de carbone, phényle ou carboalkyle ayant 2 à 7 atomes de carbone ;
n = 2,4 ;
ou d'un de ses sels pharmaceutiquement acceptables, qui comprend :
a) l'acylation d'un composé de formule : 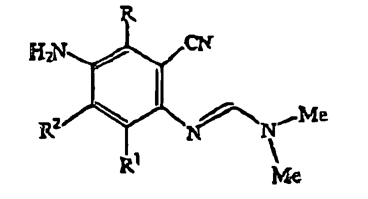 avec un halogénure d'acide ou anhydride mixte répondant à la formule : 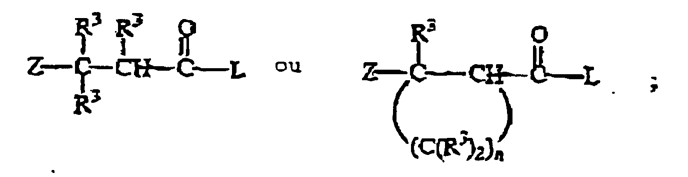 dans laquelle
Z représente un groupe -OR⁴, -SR⁴, -SOR⁴, -SO₂R⁴, halogéno, -NHR⁵ ou -NR⁵R⁵ ;
R⁴ représente un groupe alkyle ayant 1 à 6 atomes de carbone, cycloalkyle ayant 3 à 8 atomes de carbone ou phényle ;
R³ représente un groupe alkyle ayant 1 à 6 atomes de carbone ou cycloalkyle ayant 3 à 8 atomes de carbone ;
L représente un groupe Cl, Br ou -OC(O)R⁶ ;
R⁶ représente un groupe alkyle ayant 1 à 6 atomes de carbone, cycloalkyle ayant 3 à 8 atomes de carbone ou phényle ;
R³ et n répondent aux définitions précitées ;
b) la réaction du produit acylé de l'étape a) avec H₂N-X ;
dans laquelle
X répond à la définition précitée ; et
c) le traitement du composé de l'étape b) dans des conditions douces en utilisant une base ou un acide de Lewis, ce qui donne le composé de formule 1 ; et, facultativement
d) la formation d'un sel pharmaceutiquement acceptable du composé obtenu.

2. Procédé suivant la revendication 1, dans lequel l'agent d'acylation dans l'étape a) répond à la formule

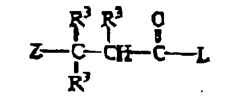

3. Procédé suivant la revendication 2, dans lequel R, R¹ et R² représentent des atomes d'hydrogène.

4. Procédé suivant la revendication 3, dans lequel X st non substitué ou est monosubstitué avec un substituant halogéno ou alkyle ayant 1 à 6 atomes de carbone.

5. Procédé suivant la revendication 4, dans lequel Z représente un groupe -OR⁴, -SR⁴, -SOR⁴, -SO²R4 ou halogéno, et R⁴ représente un groupe alkyle ayant 1 à 6 atomes de carbone.

6. Procédé suivant l'une quelconque des revendications précédentes, dans lequel les étapes a) et b) sont mises en oeuvre dans une synthèse directe en une étape.

7. Composé qui est choisi dans le groupe consistant en 1-(3-méthoxypropionyl)-3-cyano-4-(diméthylformamidyl)-aniline ; 1-(3-méthylthiopropionyl)-3-cyano-4-(diméthylformamidyl)aniline ; et 1-(3-chloropropionyl)-3-cyano-4-diméthylformamidyl)aniline.

8. Composé qui est choisi dans le groupe consistant en 4-(3-bromophényl)amine)-6-(3-méthoxypropionylamide)-quinazoline ; 4-(3-bromophénylamine)-6-(3-méthylthiopropionylamide)-quinazoline ; 4-(3-bromoprénylamins)-6-(3-méthylsulfoxydopropionylamide)-quinazoline ; et 4-(3-bromophénylamine)-6-(3-méthylsulfonopropionylamide)-quinazoline.
